(19) Europäisches Patentamt / European Patent Office / Office européen des brevets

(11) **EP 3 473 185 B1**

(12) **EUROPÄISCHE PATENTSCHRIFT**

(45) Veröffentlichungstag und Bekanntmachung des Hinweises auf die Patenterteilung:
**22.06.2022   Patentblatt 2022/25**

(21) Anmeldenummer: **18189341.3**

(22) Anmeldetag: **16.08.2018**

(51) Internationale Patentklassifikation (IPC):
**A61B 6/02** (2006.01)   **A61B 6/04** (2006.01)
**A61B 6/00** (2006.01)

(52) Gemeinsame Patentklassifikation (CPC):
**A61B 6/505; A61B 6/025; A61B 6/027;
A61B 6/0487; A61B 6/4441; A61B 6/5217;
A61B 6/5241; A61B 6/542; G16H 50/30;**
A61B 6/5205

(54) **TOMOSYNTHESEVERFAHREN UND RÖNTGENAUFNAHMEVORRICHTUNG**

TOMOSYNTHESIS METHOD AND A RADIOGRAPHIC APPARATUS

PROCÉDÉ DE TOMOSYNTHESE ET DISPOSITIF DE RADIOGRAPHIE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.10.2017   EP 17197323**

(43) Veröffentlichungstag der Anmeldung:
**24.04.2019   Patentblatt 2019/17**

(73) Patentinhaber: **Siemens Healthcare GmbH 91052 Erlangen (DE)**

(72) Erfinder:
• **Gemmel, Alexander 91056 Erlangen (DE)**
• **Kleinszig, Gerhard 91301 Forchheim (DE)**
• **Kreher, Björn 91094 Bräuningshof (DE)**
• **Swartman, Benedict 68167 Mannheim (DE)**
• **Wei, Wei 91301 Forchheim (DE)**
• **Weiten, Markus 90491 Nürnberg (DE)**
• **Yang, Qiao 90768 Fürth (DE)**

(56) Entgegenhaltungen:
**WO-A1-2009/153789      DE-A1-102015 201 067
US-A1- 2012 257 714**

**Beschreibung**

Die Erfindung betrifft ein Verfahren gemäß dem Patentanspruch 1 sowie eine Röntgenaufnahmevorrichtung zur Durchführung eines derartigen Verfahrens gemäß dem Patentanspruch 11.

Brüche des Oberschenkelknochenhalses (Oberschenkelknochen = Femur) treten relativ häufig auf und müssen in den meisten Fällen operativ behandelt werden. Bei diesem chirurgischen Eingriff wird in der Regel ein Femurnagel in den Schenkelhals eingebracht. Vor der Verriegelung des Femurnagels muss der Antetorsionswinkel zwischen dem distalen und dem proximalen Femurende ausgerichtet werden. Da die Bestimmung des Antetorsionswinkels während des chirurgischen Eingriffs sehr kompliziert ist, wird er in den meisten Fällen nur unzureichend quantifiziert. Die Ausrichtung wird nach Gefühl bzw. über einen rechts-linksvergleich der Fußstellungen durch den Arzt bestimmt. Abweichungen des Antetorsionswinkels um +/-12° sind die Regel, wodurch häufige Beschwerden und schneller Verschleiß der Gelenksflächen (Arthrose) die Folge ist.

Aus der DE 10 2015 201 067 A1 ist ein Verfahren zur Bestimmung eines Antetorsionswinkels mit einfachen zweidimensionalen Röntgenbildern bekannt.

Zu den Herausforderungen bei der Bestimmung der Antetorsionswinkels zählen die folgenden Tatsachen:

(a) Der Winkel sollte zwischen dem distalen und dem proximalen Ende des Femurs bestimmt werden und daher benötigt man eine große Aufnahmefläche (field of view). Die Femurlänge z.B. beträgt beim Erwachsenen ca. 50 cm.

(b) Da eine Verdrehung entlang der Knochenachse bestimmt werden soll, reichen einfache Projektionsbilder nicht aus. Die Bestimmung des Winkels kann bisher nur in einem Volumenbild (MPR) mit ausreichender Genauigkeit quantifiziert werden.

(c) Die Ausrichtung sollte während des chirurgischen Eingriffes durchgeführt werden und dadurch ergeben sich weitere Herausforderungen bezüglich Sterilität, Zeitknappheit und Immobilität des Patienten.

Zurzeit sind keine standarisierten Methoden zur Quantifizierung des Antetorsionswinkels während eines operativen Eingriffs bekannt.

Aus der US 2012/0257714 A1 ist ein Verfahren zur Bestimmung eines Anteversionswinkels aus Tomosynthesebilddaten bekannt. Hierfür wird mittels eines mobilen C-Bogens eine Vielzahl von sich überschneidenden Projektionsbildern entlang des Knochens aufgenommen.

Es ist Aufgabe der vorliegenden Erfindung, ein Verfahren bereitzustellen, welches die genannten Nachteile ausräumt; des Weiteren ist es Aufgabe der Erfindung, eine für die Durchführung des Verfahrens geeignete Röntgenaufnahmevorrichtung bereitzustellen.

Die Aufgabe wird erfindungsgemäß gelöst durch ein Verfahren gemäß dem Patentanspruch 1 und von einer Röntgenaufnahmevorrichtung gemäß dem Patentanspruch 11. Vorteilhafte Ausgestaltungen der Erfindung sind jeweils Gegenstand der zugehörigen Unteransprüche.

Das erfindungsgemäße Verfahren zur Bestimmung einer Ausrichtung zwischen zumindest zwei Knochenteilen eines langgestreckten Knochensystems eines Patienten umfasst die folgenden Schritte:

- Aufnahme von einer Vielzahl von sich teilweise räumlich überlagernden Projektionsbildern durch ein Aufnahmesystem eines Röntgengeräts, insbesondere eines mobilen Röntgengeräts, während einer Translationsbewegung des Röntgengeräts oder des Aufnahmesystems in Richtung einer oder parallel zu einer Longitudinalachse des Knochensystems,
- Rekonstruktion von Tomosynthesebilddaten, insbesondere von Schichtbildern, der Knochenteile aus den aufgenommenen Projektionsbildern, und
- Bestimmung oder Schätzung eines Ausrichtungswinkels zwischen den zumindest zwei Knochenteilen zumindest teilweise anhand der Projektionsbilder oder der rekonstruierten Tomosynthesebilddaten.

Durch das erfindungsgemäße Verfahren werden vorteilhaft die auftretenden Probleme für die Bestimmung eines Ausrichtungswinkels, z.B. Antetorsionswinkels, eines langgestreckten Knochensystems, z.B. Femurs, behoben. Durch die Translationsbewegung des mobilen Röntgengeräts kann ein besonders "langes" field of view abgebildet werden, die Länge des bei Bedarf resultierenden Schichtvolumens kann durch die Länge des gefahrenen Weges nach Bedarf bestimmt werden. Diese Länge kann z.B. vom operierenden Arzt definiert werden. Durch die Tomosynthese können nicht nur zweidimensionale Bilder aufgenommen, sondern auch dreidimensionale Schichtbilder bzw. dreidimensionale Teilbilder, also Volumendarstellungen aufgenommen und rekonstruiert werden. Durch diese dreidimensionalen Schichtbilder oder Teilbilder ist eine Quantifizierung des Ausrichtungswinkels, z.B. Antetorsionswinkels, auf einfache Weise und in guter Qualität möglich. Insbesondere ein mobiles Röntgengerät kann zudem in einem sterilen Umfeld, also während einer Operation, eingesetzt werden. Die translatorische Bewegung des mobilen Röntgengerätes kann mittels eines sehr schnellen, einfachen und aufwandsarmen Workflows erzielt werden. Die notwendige Strahlendosis ist für eine Tomosyntheseaufnahme deutlich geringer als für übliche Volumenscans wie CT oder DynaCT.

Durch das Verfahren kann z.B. der Antetorsionswinkel während eines chirurgischen Eingriffes bestimmt werden, bevor ein benötigter Femurnagel verriegelt wird, so dass bei Feststellung eines nicht passenden Antetorsionswinkel eine Korrektur ermöglicht wird. Im Rahmen der Korrektur kann das distale und das proximale Ende des Femurs anschließend genauer zueinander ausgerichtet werden. Die Korrekturmöglichkeit resultiert in besseren Operationsergebnissen und weniger Komplikationen und Beschwerden bei Patienten.

Die Bestimmung bzw. Schätzung des Ausrichtungswinkels wird zumindest teilweise auf der Basis von rekonstruierten Tomosynthesebilddaten, also z.B. eines rekonstruierten Schichtbildes oder rekonstruierter Teilbilder oder Bildausschnitte durchgeführt. Bei Vorliegen eines Schichtbildes oder von zueinander registrierten Teilbildern oder Bildausschnitten können z.B. mittels Segmentierung oder Objekterkennung die zumindest zwei Knochenteile des langgestreckten Knochensystems erkannt werden. Anschließend kann z.B. durch Anlegen von Achsen oder Tangenten an die relevanten Strukturen der zumindest zwei Knochenteile der zwischen den zwei Knochenteilen auftretende Ausrichtungswinkel bestimmt bzw. geschätzt werden. Das Ergebnis kann dann z.B. mittels Anzeigevorrichtung ausgegeben werden. Beim Antetorsionswinkel handelt es sich um den Winkel zwischen dem Femurhals und den Femurkondylen, genauer gesagt um den Winkel, der von einer Femurhalsachse und einer Femurkondylentangente gebildet wird. Bei Vorliegen eines dreidimensionalen Schichtbildes, wie im vorliegenden Verfahren erstellt, kann auf einfache Weise der Antetorsionswinkel bestimmt werden.

Alternativ zu einem Femur und einem Antetorsionswinkel kann auch das langgestreckte Knochensystem von einem Wirbelsäulenabschnitt und der Ausrichtungswinkel von einem Winkel zwischen zwei Wirbelkörpern gebildet werden. Wirbelsäulenverletzungen treten bei fast 28% aller schwerverletzten Patienten in Deutschland auf. Statistisch gesehen sind davon bei jedem 6. Patienten die unteren Halswirbel betroffen. Da diese Eingriffe sehr anspruchsvoll sind, ist der Chirurg während des Eingriffes auf bildgebende Verfahren angewiesen. Allerdings ist die intraoperative 2D-Bildgebung wie die konventionelle Radiographie im cervicothorakalen Übergang aufgrund starker Überlagerungen der Schultern limitiert und eine ausreichend genaue Darstellung eines Repositionsergebnisses der Wirbelsäule nur mit einer 3D Bildgebung möglich. Durch das erfindungsgemäße Verfahren können auch hier alle Probleme einfach gelöst werden. Es kann im Operationsraum auf schnelle und einfache Weise der langgezogene interessierende Bereich als dreidimensionales Schichtbild oder Ausschnitte davon als 3D Teilbilder dargestellt werden, wobei die Strahlenbelastung für den Patienten gering bleibt. Wird ein nicht optimaler Winkel zwischen z.B. zwei Wirbelkörpern ermittelt bzw. festgestellt, kann ein weiterer Eingriff zur Korrektur durchgeführt werden.

Nach einer weiteren Ausgestaltung der Erfindung wird der Ausrichtungswinkel zwischen den zumindest zwei Knochenteilen ausschließlich anhand von rekonstruierten Tomosynthesebilddaten, insbesondere Schichtbildern, bestimmt oder geschätzt.

Nach einer weiteren Ausgestaltung der Erfindung werden zur Bestimmung oder Schätzung des Ausrichtungswinkels zwischen den zumindest zwei Knochenteilen weitere Projektionsbilder aus unterschiedlichen Projektionsrichtungen, z.B. laterale Projektionsbilder, verwendet werden.

Nach einer weiteren Ausgestaltung der Erfindung erfolgt während der Aufnahme und der Translationsbewegung des Röntgengeräts zusätzlich eine Rotationsbewegung des Aufnahmesystems. Durch diese kann die 3D-Bildgebung signifikant verbessert werden. Es kann hier z.B. eine Rotation von z.B. 60°, also z.B. Rotationswinkel von +30° bis -30°, vorgesehen sein.

Nach einer Ausgestaltung der Erfindung ist der Abstand zwischen je zwei Bildern der Vielzahl von sich teilweise räumlich überlagernden Bildern kleiner oder gleich der halben durch einen Öffnungswinkel $\alpha$ einer Röntgenquelle des Aufnahmesystems erfassbaren Objektbreite (d'). Ein Abstand kleiner oder gleich der halben erfassbaren Objektbreite d' liefert genügend Tiefeninformationen für ein hochqualitatives dreidimensionales Schichtbild. Die erfassbare Objektbreite d' ist der Ausschnitt aus dem langgezogenen Knochensystem, welcher maximal auf die tatsächliche Detektorbreite d des Röntgendetektors des Aufnahmesystems projiziert werden kann. Es gilt der Zusammenhang $d' = d\frac{SOD}{SID}$, wobei SOD (Source Object Distance) der Abstand zwischen dem Objekt und der Röntgenquelle ist und SID (Source Image Distance) der Abstand zwischen der Röntgenquelle und dem Röntgendetektor des Aufnahmesystems.

Erfindungsgemäß unterscheidet sich der Abstand zwischen je zwei Bildern der Vielzahl von sich teilweise räumlich überlagernden Bildern abhängig von einer Aufnahmeposition entlang des langgestreckten Knochensystems derart, dass relevante Bereiche mit kleinem Abstand und weniger relevante mit größerem Abstand aufgenommen werden. So kann z.B. bei einem Femur vorgesehen sein, dass der Abstand im zentralen Bereich des langgestreckten Knochensystems, also z.B. des Femurschaftes, höher ist und somit die Bildqualität geringer, da hier keine relevanten Bereiche für die Bestimmung des Antetorsionswinkels vorhanden sind. Es kann bei anderen langgestreckten Knochensystemen auch vorgesehen sein, dass im Bereich einer Fraktur bzw. im Berührungsbereich der zwei Knochenteile der Abstand geringer ist und somit dort eine höhere Bildqualität erzielt werden kann.

Nach einer weiteren Ausgestaltung der Erfindung erfolgt die Translationsbewegung mittels insbesondere automatisch angesteuerter Rollen des mobilen Röntgengeräts entlang eines Fußbodens. Die motorisierten Rollen oder Räder können mittels der Systemsteuerung angesteuert werden. Alternativ kann ein festinstalliertes Röntgengerät auf Schienen verfahren werden oder es kann lediglich das Aufnahmesystem (C-Bogen) eines festinstallierten Röntgengeräts an seiner Aufhängung translatorisch bewegt werden.

Die Erfindung umfasst eine Röntgenaufnahmevorrichtung zur Durchführung des Verfahrens, welche als mobiles C-Bogen-Röntgengerät ausgebildet ist, aufweisend ein an einem C-Bogen gehaltertes Aufnahmesystem mit einer Röntgenquelle und einem Röntgendetektor, eine Systemsteuerung zur Ansteuerung der Aufnahme von einer Vielzahl von sich teilweise räumlich überlagernden Bildern, insbesondere Tomosynthesebilddaten, durch das Aufnahmesystem während einer Translationsbewegung des Aufnahmesystems des Röntgengeräts, und eine Bildbearbeitungseinheit und eine Berechnungseinheit zur Rekonstruktion von Schichtbildern und Bestimmung des Ausrichtungswinkels. In vorteilhafter Weise weist die Röntgenaufnahmevorrichtung einen auf Rollen automatisch verfahrbaren Gerätewagen auf, an welchem der C-Bogen angeordnet ist.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:

FIG 1     ein bekanntes Aufnahmesystem zur Aufnahme eines Femurausschnitts;

FIG 2     eine Schnittansicht eines bekannten Antetorsionswinkels;

FIG 3     eine Draufsicht eines bekannten Antetorsionswinkels;

FIG 4     ein Aufnahmesystem zur Aufnahme einer Vielzahl von sich teilweise räumlich überlagernden Bildern zur Darstellung eines Femurs;

FIG 5     eine vergrößerte Ansicht der Aufnahmegeometrie gemäß FIG 4 mit drei verschiedenen Projektionsrichtungen zur Rekonstruktion eines Schichtbildes;

FIG 6     eine weitere Ansicht der Aufnahmegeometrie gemäß FIG 4;

FIG 7     ein Aufnahmesystem zur Aufnahme einer Vielzahl von sich teilweise räumlich überlagernden Bildern zur Darstellung einer Wirbelsäule;

FIG 8     ein erfindungsgemäßes mobiles C-Bogen-Röntgengerät; und

FIG 9     eine Abfolge der Schritte des erfindungsgemäßen Verfahrens;

FIG 10     ein Aufnahmesystem mit gleichzeitiger Translations- und Rotationsbewegung.

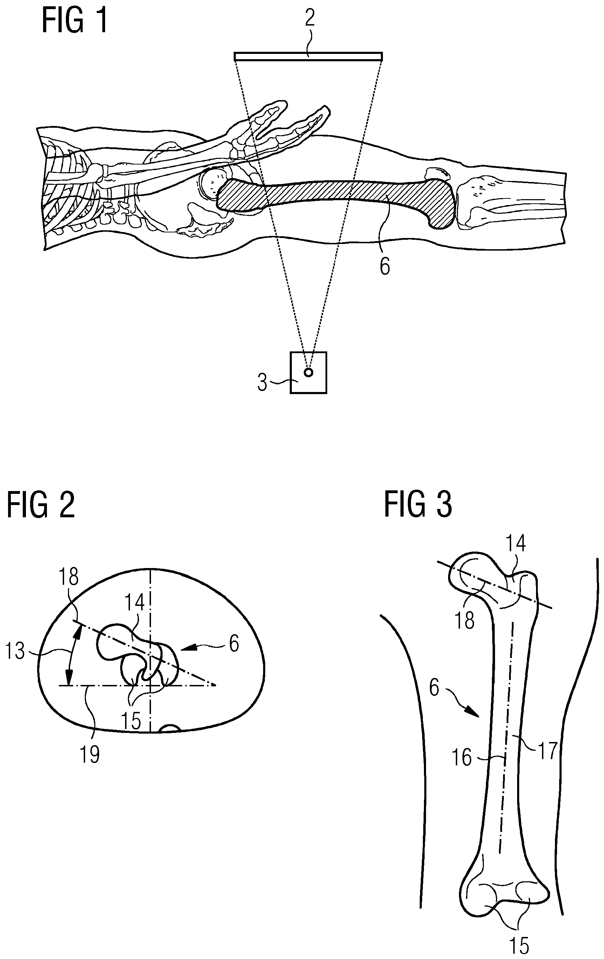

In der FIG 1 ist ein bekanntes Aufnahmesystem mit einem Röntgendetektor 2 und einer Röntgenquelle 3 zur Aufnahme eines zweidimensionalen Röntgenbilds eines Femurs gezeigt. Mittels eines derartigen Bildes ist es nur sehr beschränkt möglich, nach einer Fraktur und während einer OP zur Korrektur des Femurs 6 einen Antetorsionswinkel zu bestimmen. Es ist denkbar, mithilfe von mobilen 3D-fähigen Röntgengeräten ein Volumen am distalen Femurende und eines am proximalen Femurende zu akquirieren. Auf Grund des komplexen Workflows und der hohen applizierten Strahlendosis wird dies jedoch nicht durchgeführt und würde auch nicht zu einer optimalen Bestimmung des Antetorsionswinkels führen, da zwei unabhängige Volumina ausgewertet werden müssen.

Zur Erläuterung ist in den Figuren 2 und 3 als Schnittbild und als Draufsicht ein bekannter Antetorsionswinkel 13 gezeigt. Es handelt sich dabei um den Winkel zwischen dem Femurhals 14 und den Femurkondylen 15, genau gesagt um den Winkel, der von der Femurhalsachse 7 und der Femurkondylentangente 19 gebildet wird. Häufig sind nach einem Bruch des Femurs 6 und einer unvollständigen Korrektur der Femurhals 14 und die Femurkondylen 15 entlang der Femurschaftachse 16 des Femurschaftes 17 verdreht zueinander angeordnet.

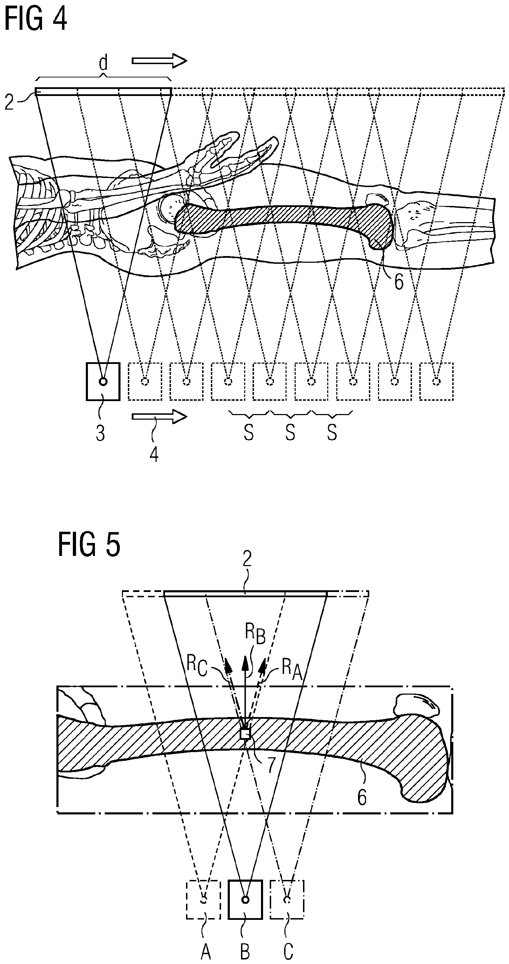

In der FIG 4 ist ein Aufnahmesystem zur Aufnahme einer Vielzahl von sich teilweise räumlich überlagernden Bildern zur Darstellung eines Femurs gezeigt. Das Aufnahmesystem wird hierfür im Wesentlichen in Richtung 4 des langgestreckten Knochensystems, z.B. der Femurschaftachse des Femurs, bzw. parallel dazu in einer Translationsbewegung bewegt und währenddessen werden in im Allgemeinen regelmäßigen Abständen Röntgenbilder aufgenommen. Die Bewegungsabfolge ist dabei als Vielzahl von Röntgenquelle 3 - Röntgendetektor 2 - Kombinationen dargestellt, wobei in den dargestellten Positionen die jeweiligen Röntgenbilder aufgenommen werden. Der Quellenabstand s ist dabei der Abstand zwischen je zwei Röntgenbildern. Unter der Annahme, dass der Source-Objekt Abstand (SOD; Abstand zwischen der Röntgenquelle und dem Aufnahmeobjekt, also langgestreckten Knochensystem) entlang der verfahrenen Trajektorie konstant bleibt, entspricht für einen bestimmten SID (Source-Image Distance; Abstand zwischen Röntgendetektor und Röntgenquelle) die erfassbare Objektbreite $d' = d*SOD/SID$, wobei d die tatsächliche Detektorbreite ist.

In der FIG 5 ist ein Ausschnitt aus der Darstellung aus FIG 4 gezeigt, wobei hier die Geometrie bei drei abfolgenden Röntgenbildern gezeigt ist. Auf Grund der Geometrie werden vollständig überfahrene Raumpunkte 7 in den Röntgenbildern aus unterschiedlichen Projektionsrichtungen dargestellt. Dies kann verwendet werden, um mithilfe eines Tomosyntheseverfahrens ein Schichtbild des vollständig überfahrenden Bereichs zu rekonstruieren. In einer ersten Position A des Aufnahmesystems wird der Raumpunkt 7 aus der ersten Projektionsrichtung $R_A$ abgebildet, in einer zweiten Position B des Aufnahmesystems aus der zweiten Projektionsrichtung $R_B$ und in einer dritten Position C des Aufnahmesystems aus der dritten Projektionsrichtung $R_C$. Dies wird verwendet, um mithilfe eines Tomosyntheseverfahrens ein Schichtbild zu rekonstruieren.

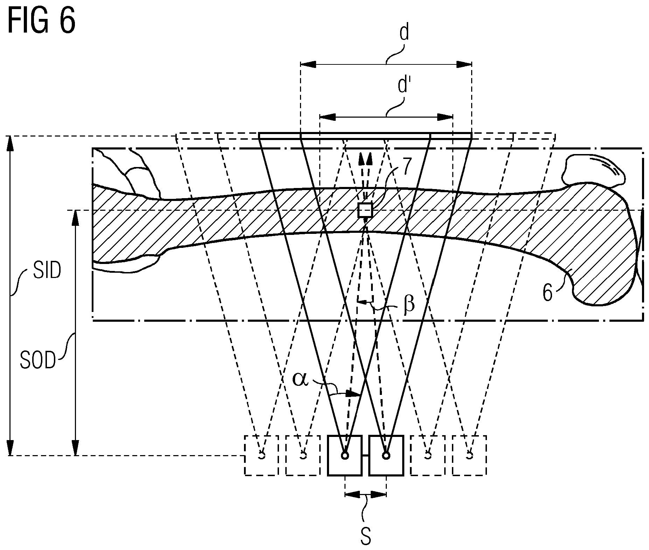

Um aus zweidimensionalen Röntgenbildern Tiefeninformationen zu erhalten, müssen die relevanten anatomischen Positionen auf mindestens je zwei Röntgenbildern dargestellt sein. Als Konsequenz muss der Quellenabstand s zwischen je zwei Röntgenaufnahmen kleiner oder gleich der Hälfte der erfassbaren Objektbreite d' sein (siehe FIG 6). In diesem Fall kann der Winkel $\beta$ zwischen je zwei Projektionsrichtungen mit

$$\beta \approx \frac{1}{2}\alpha$$

angenähert werden, wobei $\alpha$ dem Öffnungswinkel der Röntgenquelle entspricht und außerdem gilt: $\tan \alpha = d/SID$.

Die Genauigkeit der Antetorsionswinkelbestimmung hängt stark davon ab, wie exakt 3D Informationen aus den Röntgenbildern bestimmt werden können. Eine fehlerhafte Verschiebung $\Delta$ innerhalb der Bildebene geht mit einen Effekt von $\Delta/\tan \beta$ in die Schätzung des Abstandes zwischen Röntgenquelle und Untersuchungsobjekt (SOD) ein. Daher muss gerade bei kleinen Öffnungswinkeln $\alpha$ darauf geachtet werden, dass der Winkel $\beta$ maximiert wird, um die Genauigkeit der Antetorsionswinkelbestimmung zu verbessern.

Es kann gezeigt werden, dass bei einem Quellenabstand zwischen den Aufnahmen von

$$s(n) = \frac{1}{2}^n d'$$ der Winkel $\beta$ zwischen den Projektionsrichtungen

$$\beta(n) \approx \alpha\left(1 - \frac{1}{2}^n\right)$$

entspricht, wobei n die Anzahl der Projektionsrichtungen pro Raumpunkt ist. Das heißt, dass mit zunehmenden n sich der maximale Winkel zwischen den Projektionen an $\alpha$ annähert. Bei ausreichendem SNR der Röntgenbilder ist für die Bestimmung des Antetorsionswinkels n>3 nicht notwendigerweise sinnvoll, da die Erhöhung von $\beta$ nur noch gering ausfällt, $\beta(3) \approx 0.9 \cdot \alpha$.

Erfindungsgemäß unterscheidet sich der Abstand zwischen je zwei Bildern der Vielzahl von sich teilweise räumlich überlagernden Bildern abhängig von einer Aufnahmeposition entlang des langgestreckten Knochensystems. So kann z.B. bei dem Femur vorgesehen sein, dass der Abstand im zentralen Bereich des Femurschaftes höher ist und somit dort eine geringere Bildqualität erzielt werden kann.

Dieses sogenannte adaptive Sampling kann durchgeführt werden, um Dosis zu sparen. Im Bereich des Schaftes befinden sich keine anatomischen Strukturen, die für die Bestimmung des Antetorsionswinkels relevant sind. Hier kann der Abstand der Röntgenbilder auf s(1)=1/2 d' reduziert werden. Bei anderen langgestreckten Knochensystemen werden ebenfalls relevante Bereiche mit besonders hoher Qualität (kleiner Abstand) und weniger relevante mit geringerer Qualität (größerer Abstand) aufgenommen.

Zur weiteren Dosisreduktion können die Bereiche, die nicht zum Femur gehören durch einen Kollimator weggeblendet werden.

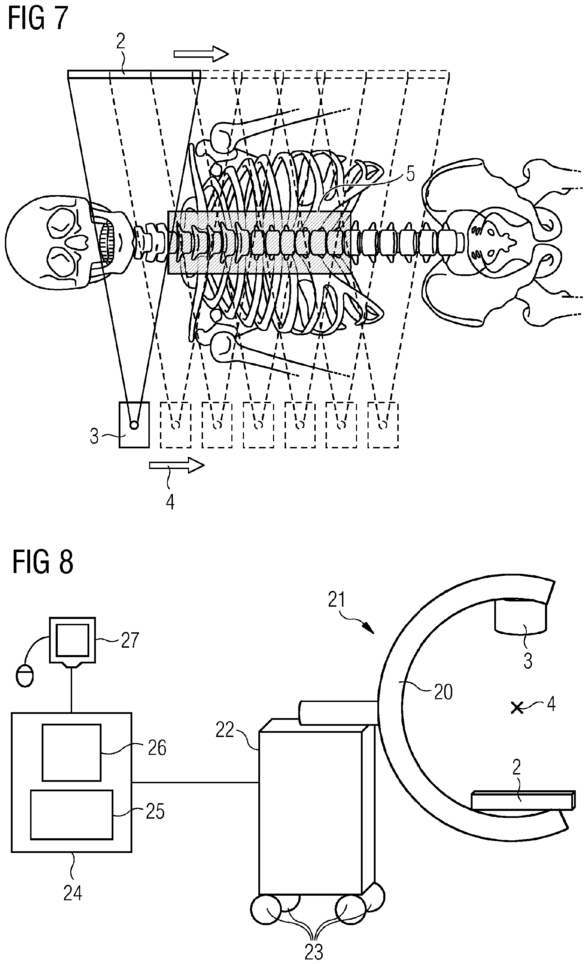

In der FIG 7 ist als weiteres Anwendungsbeispiel eine Darstellung der Wirbelsäule gezeigt. Hier kann mittels des Verfahrens ein großer Teil 5 einer Wirbelsäule abgebildet und ein Winkel zwischen zwei Wirbelkörpern bestimmt werden. Auf Grund der starken Überlagerung des Schulterbereichs im cervicothorakalen Übergang ist eine ausreichend genaue Darstellung eines Repositionsergebnisses während einer OP der Wirbelsäule nur mit 3D Bildgebung möglich. Beim bekannten 3D-Scan muss sich die Röntgenvorrichtung um den Patienten herumbewegen. Das erfindungsgemäße Verfahren ermöglicht eine zügige Kontrolle des Repositionsergebnisses durch Abbildung des Wirbelkörperalignments in der sagittalen Ebene. Sie erlaubt eine scharfe Abbildung einer in der Tiefe liegenden Schicht unabhängig von überlagernden Strukturen wie z.B. den Schultern. Im Vergleich zum bekannten 3D-Scan ist die Technik schneller, aufgrund fehlender orbitaler Rotation besser integrierbar und erfordert weniger Strahlenbelastung. Es kann bei Bedarf die gesamte Wirbelsäule zusammenhängend abgebildet werden. Durch die Wahl der richtigen Schicht (Schichtposition und Schichtdicke) kann die Wirbelsäule isoliert von überlagernden Objekten (wie z.B. Dem Schulterbereich) dargestellt werden.

Ein hierfür geeignetes mobiles C-Bogen-Röntgengerät 21 ist in der FIG 8 gezeigt. Es weist einen C-Bogen 20 auf, welches eine Röntgenquelle 3 und einen Röntgendetektor 2 haltert. Diese bilden das Aufnahmesystem. Das mobile C-Bogen-Röntgensystem 21 weist einen Gerätewagen 22 auf, welcher auf Rollen 23 verfahrbar ist und an welchem der C-Bogen 20 angeordnet ist. Die Rollen 23 sind ansteuerbar und können motorisiert verfahren werden, z.B. mit einer definierten Geschwindigkeit in eine definierte Richtung. Das C-Bogen-Röntgengerät 21 wird von einer Systemsteuerung 24 angesteuert. Diese kann z.B. eine Aufnahme von einer Vielzahl von sich teilweise räumlich überlagernden Röntgenbildern, insbesondere Tomosynthesebilddaten, ansteuern, während sich das mobile C-Bogen-Röntgengerät 21 mittels des Gerätewagens 22 in einer definierten Translationsbewegung bewegt. Außerdem weist das C-Bogen-Röntgensystem eine Bildbearbeitungseinheit 25 zur Bearbeitung von Röntgenbildern und eine Berechnungseinheit 26 zur Rekonstruktion von Schichtbildern aus der Vielzahl von Röntgenbildern und zur Bestimmung des Ausrichtungswinkels aus den Schichtbildern auf. Ein solches C-Bogen-Röntgengerät 21 ist in einem sterilen Umfeld und während eines chirurgischen Eingriffs einsetzbar.

Das langgestreckte Knochensystem, z.B. Femur, wird so nah wie mit der Patientensicherheit vereinbar an dem Röntgendetektor 2 angeordnet.

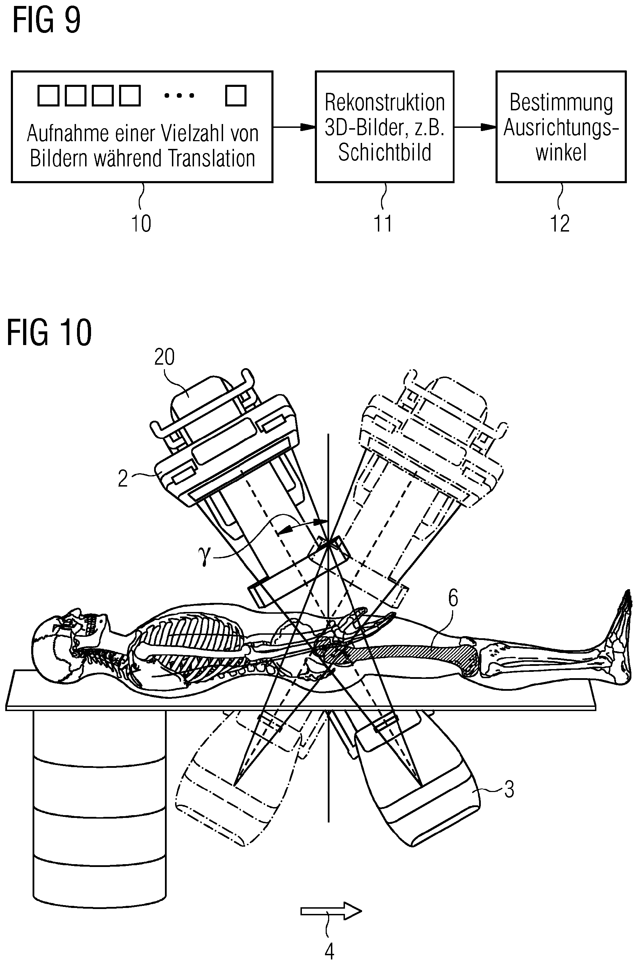

In der FIG 9 ist eine Abfolge der Schritte des erfindungsgemäßen Verfahrens gezeigt. In einem ersten Schritt 10 wird eine Vielzahl von sich teilweise räumlich überlagernden Röntgenbildern aufgenommen, während sich das Aufnahmesystem (Röntgenquelle und Röntgendetektor bzw. der diese halternde C-Bogen) des insbesondere mobilen C-Bogen-Röntgengeräts 21 in einer Translationsbewegung in Richtung der oder parallel zu der Longitudinalachse des langgestreckten Knochensystems (z.B. Femur oder Wirbelsäule) bewegt. Im Falle des mobilen C-Bogen-Röntgengeräts bewegt sich das vollständige C-Bogen-Röntgengerät auf dem Gerätewagen mittels der automatisch angesteuerten Rollen in einer definierten Translationsbewegung derart vorwärts, dass der Quellenabstand s zwischen je zwei Bildern kleiner oder gleich der halben Detektorbreite des Röntgendetektors beträgt. Auf Grund der Röntgenquelle-Röntgendetektor-Geometrie (sich auffächernder Röntgenstrahl) werden dabei die vollständig überfahrenen Raumpunkte 7 in der Vielzahl von Röntgenbildern aus unterschiedlichen Projektionsrichtungen dargestellt. Es ist entsprechend vorteilhaft, die jeweiligen einzelnen Röntgenbilder in einer möglichst kurzen Zeit aufzunehmen, z.B. in weniger als 5 ms pro Röntgenbild, damit es zu möglichst wenig Verschmierung kommt.

Alternativ kann ein nicht-mobiles C-Bogen-Röntgengerät auch auf Schienen verschiebbar sein oder es kann lediglich das Aufnahmesystem (C-Bogen) eines festinstallierten Röntgengeräts bewegt werden.

In einem zweiten Schritt 11 wird aus den aufgenommenen sich teilweise räumlich überlagernden Röntgenbildern z.B. ein dreidimensionales Schichtbild der Knochenteile des langgestreckten Knochensystems rekonstruiert, also z.B. der gesamte Femur eines Patienten. Alternativ können auch nur 3D-Teilbilder bzw. Ausschnitte aus Bildern rekonstruiert werden, also z.B. nur der Femurkopf und/oder die Femurkondylen. In einem dritten Schritt 12 wird ein Ausrichtungswinkel zwischen den zumindest zwei Knochenteilen zumindest teilweise anhand des/der rekonstruierten Schichtbilder oder der Teilbilder oder der Projektionsbilder bestimmt oder geschätzt. Da durch das Tomosyntheseverfahren ein vollständiges Schichtbild des gesamten langgestreckten Knochensystems erstellt werden kann, sind die geometrischen Zusammenhänge des Knochensystems aus diesem einen Volumenbild auf einfache Weise ableitbar, z.B. unter Anlegen von Geraden und Tangenten an die relevanten Strukturen und/oder Verwendung von Segmentierungen und/oder Bilderkennungsalgorithmen.

Das erfindungsgemäße Verfahren ermöglicht, z.B. den Antetorsionswinkel während eines chirurgischen Eingriffes zu bestimmen, bevor der Femurnagel verriegelt wird. Das Verfahren zeichnet sich aus durch einen einfachen Workflow innerhalb einer sterilen Umgebung, ein frei definierbares field of view entlang der Bewegungsrichtung und resultierende Schichtbilder zur Bestimmung z.B. des Antetorsionswinkels. Durch die Bestimmung des Antetorsionswinkels kann das distale und das proximale Ende des Femurs genauer zueinander ausgerichtet werden. Dies resultiert in weniger Komplikationen und Beschwerden von Patienten.

Das erfindungsgemäße Verfahren hat außerdem den Vorteil, dass auf Grund der translatorischen Bewegung der Workflow für die Bildakquisition sehr einfach ist. Die Komplexität für den Anwender, z.B. einen chirurgischen Eingriff durchführenden Arzt ist reduziert, da er den Ausrichtungswinkel (z.B. Antetorsionswinkel) anhand eines vollständigen Volumens quantifizieren kann und dafür nicht z.B. zwei oder mehr unabhängig voneinander erstellte Volumenbilder benötigt. Zusätzlich ist die applizierte Strahlendosis für einen Tomosynthese-Scan deutlich geringer als z.B. für zwei 3D Aufnahmen.

In der FIG 10 ist als weiteres Ausführungsbeispiel ein Verfahren gezeigt, bei welchem zusätzlich zur Translation auch eine Rotation des C-Bogens erfolgt, um einen Antetorsionswinkel zu bestimmen. Im Folgenden wird der dazu gehörige Workflow beschrieben. Das Bein des Patienten sollte während des gesamten Workflows nicht bewegt werden.

In einem ersten Teil des Workflows wird die Orientierung des distalen Femurendes gesucht. Der C-Bogen wird lateral angeordnet (nicht gezeigt) und es wird manuell per Fluoroskopie versucht, eine Projektionsansicht zu finden, bei der der mittlere und der laterale Kondylus entlang des Röntgenstrahls hintereinander angeordnet sind und eine gemeinsame Unterkante bilden. Die Unterkante wird dann manuell bestimmt. Daraus werden die Orientierung und die Höhe des distalen Femurendes bzw. die Femurkondylentangente berechnet.

Der zweite und der dritte Teil des Workflows werden von einer Ausgestaltung des erfindungsgemäßen Verfahrens gebildet.

Im zweiten Teil des Workflows wird der C-Bogen 20 so positioniert, dass er eine Translation in Richtung 4 entlang des Femurs 6, insbesondere umfassend Femurkopf und großen Trochanter, durchführt. Außerdem wird er um einen Rotationswinkel γ gekippt, z.B. um -30° oder soweit es ohne Kollision mit dem Patienten möglich ist. Während Aufnahme der Vielzahl von Projektionsbildern in hoher Frequenz wird der C-Bogen 20 in Richtung 4 der Achse des langgestreckten Femurs 6 translatiert und dabei gleichzeitig rotiert, z.B. zwischen den Rotationswinkeln +30° und -30° (also über einen Winkelinkrement von 60°) bzw. zwischen dem maximal und dem minimal möglichen Rotationswinkel γ (dies kann sich auch während der Translation ändern, der Winkelinkrement z.B. zwischen 60° und 78° schwanken). Währenddessen wird der Zusammenhang zwischen Rotation und Translation berechnet bzw. detektiert. Insbesondere werden so der Femurkopf und der große Trochanter abgetastet, es kann auch der gesamte Femur abgetastet werden.

Im dritten Teil des Workflows erfolgt die Rekonstruktion und Berechnung des Antetorsionswinkels. Anhand der Vielzahl von aufgenommenen Projektionsbildern wird ein 3D-Teilbild der Oberfläche des Femurkopfes und des großen Trochanters rekonstruiert. Auf diesen Bilddaten basierend wird die Orientierung der Femurhalsachse bestimmt. Der Antetorsionswinkel ergibt sich dann aus der Differenz der Orientierungen von Femurhalsachse und zuvor bestimmter Femurkondylentangente.

Die Erfindung lässt sich in folgender Weise kurz zusammenfassen: Die Erfindung umfasst ein Verfahren zur Bestimmung einer Ausrichtung zwischen zumindest zwei Knochenteilen eines langgestreckten Knochensystems eines Patienten mit den Schritten: Aufnahme von einer Vielzahl von sich teilweise räumlich überlagernden Projektionsbildern durch ein Aufnahmesystem eines Röntgengeräts, insbesondere eines mobilen Röntgengeräts, während einer Translationsbewegung des Röntgengeräts oder des Aufnahmesystems in Richtung einer oder parallel zu einer Longitudinalachse des Knochensystems, Rekonstruktion von Tomosynthesebilddaten, insbesondere von Schichtbildern, der Knochenteile aus den aufgenommenen Projektionsbildern, und Bestimmung oder Schätzung eines Ausrichtungswinkels zwischen den zumindest zwei Knochenteilen zumindest teilweise anhand der rekonstruierten Tomosynthesebilddaten und/oder der Projektionsbilder.

**Patentansprüche**

1. Verfahren zur Bestimmung einer Ausrichtung zwischen zumindest zwei Knochenteilen eines langgestreckten Knochensystems eines Patienten mit den folgenden Schritten:

   • Aufnahme von einer Vielzahl von sich teilweise räumlich überlagernden Projektionsbildern durch ein Aufnahmesystem eines Röntgengeräts, insbesondere eines mobilen Röntgengeräts, während einer Translationsbewegung des Röntgengeräts oder des Aufnahmesystems in Richtung einer oder parallel zu einer Longitudinalachse des Knochensystems (Schritt 10), wobei sich der Abstand zwischen je zwei Bildern der Vielzahl von sich teilweise räumlich überlagernden Projektionsbildern abhängig von einer Aufnahmeposition entlang des langgestreckten Knochensystems unterscheidet, indem relevante Bereiche mit kleinem Abstand und weniger relevante mit größerem Abstand aufgenommen werden,

   • Rekonstruktion von Tomosynthesebilddaten, insbesondere von Schichtbildern, der Knochenteile aus den aufgenommenen Projektionsbildern (Schritt 11), und
   • Bestimmung oder Schätzung eines Ausrichtungswinkels zwischen den zumindest zwei Knochenteilen zumindest teilweise anhand der rekonstruierten Tomosynthesebilddaten und/oder der Projektionsbilder (Schritt 12).

2. Verfahren nach Anspruch 1, welches mittels eines mobilen C-Bogen Röntgengeräts ausgeführt wird.

3. Verfahren nach einem der vorangehenden Ansprüche, wobei während der Aufnahme und der Translationsbewegung des Röntgengeräts zusätzlich eine Rotationsbewegung des Aufnahmesystems erfolgt.

4. Verfahren nach einem der vorangehenden Ansprüche, wobei der Ausrichtungswinkel zwischen den zumindest zwei Knochenteilen ausschließlich anhand von rekonstruierten Tomosynthesebilddaten, insbesondere Schichtbildern, bestimmt oder geschätzt wird.

5. Verfahren nach einem der Ansprüche 1 bis 3, wobei zur Bestimmung oder Schätzung des Ausrichtungswinkels zwischen den zumindest zwei Knochenteilen weitere Projektionsbilder verwendet werden.

6. Verfahren nach einem der vorangehenden Ansprüche, wobei der Abstand (s) zwischen je zwei Bildern der Vielzahl von sich teilweise räumlich überlagernden Projektionsbildern kleiner oder gleich der halben durch einen Öffnungswinkel $\alpha$ einer Röntgenquelle (3) des Aufnahmesystems erfassbaren Objektbreite (d') beträgt.

7. Verfahren nach einem der vorangehenden Ansprüche, wobei das langgestreckte Knochensystem von einem Femur (6) und der Ausrichtungswinkel von einem Antetorsionswinkel (13) gebildet werden.

8. Verfahren nach einem der vorangehenden Ansprüche, wobei das langgestreckte Knochensystem von einem Wirbelsäulenabschnitt (8) und der Ausrichtungswinkel von einem Winkel zwischen zwei Wirbelkörpern gebildet werden.

9. Verfahren nach einem der vorangehenden Ansprüche, wobei die Translationsbewegung mittels, insbesondere automatisch angesteuerter, Rollen (23) des mobilen Röntgengeräts entlang eines Fußbodens erfolgt.

10. Verfahren nach einem der vorangehenden Ansprüche, wobei die Translationsbewegung entlang zumindest einer Schiene erfolgt.

**EP 3 473 185 B1**

**11.** Röntgenaufnahmevorrichtung zur Durchführung eines Verfahrens nach einem der Ansprüche 1 bis 9, welche insbesondere als mobiles C-Bogen-Röntgengerät (21) ausgebildet ist, aufweisend

• ein an einem C-Bogen (20) gehaltertes Aufnahmesystem mit einer Röntgenquelle (3) und einem Röntgendetektor (2),
• eine Systemsteuerung (24) zur Ansteuerung der Aufnahme von einer Vielzahl von sich teilweise räumlich überlagernden Projektionsbildern durch das Aufnahmesystem während einer Translationsbewegung des Röntgengeräts, wobei sich der Abstand zwischen je zwei Bildern der Vielzahl von sich teilweise räumlich überlagernden Projektionsbildern abhängig von einer Aufnahmeposition entlang des langgestreckten Knochensystems unterscheidet, indem relevante Bereiche mit kleinem Abstand und weniger relevante mit größerem Abstand aufgenommen werden, und
• eine Bildbearbeitungseinheit (25) und eine Berechnungseinheit (26) zur Rekonstruktion von Tomosynthesebilddaten, insbesondere Schichtbildern, und Bestimmung des Ausrichtungswinkels.

**12.** Röntgenaufnahmevorrichtung nach Anspruch 11, welche als mobiles C-Bogen-Röntgengerät (21) ausgebildet ist, aufweisend einen auf Rollen (23) automatisch verfahrbaren Gerätewagen (22), an welchem der C-Bogen (20) angeordnet ist.

**Claims**

**1.** Method for determining an alignment between at least two bone parts of an elongated bone system of a patient with the following steps:

• Recording a plurality of partially spatially overlapping projection images by means of a recording system of an x-ray device, in particular a mobile x-ray device, during a translational movement of the x-ray device or the recording system in the direction of or parallel to a longitudinal axis of the bone system (step 10), wherein the distance between two images of the plurality of partially spatially overlapping projection images differs as a function of a recording position along the elongated bone system by relevant regions being recorded with a small distance and less relevant with a larger distance,
• Reconstructing tomosynthesis image data, in particular of slice images, of the bone parts from the recorded projection images (step 11) and
• Determining or estimating an alignment angle between the at least two bone parts at least partially on the basis of the reconstructed tomosynthesis image data and/or the projection images (step 12).

**2.** Method according to claim 1, which is carried out by means of a mobile C-arm x-ray device.

**3.** Method according to one of the preceding claims, wherein a rotational movement of the recording system is carried out during the recording and the translational movement of the x-ray device.

**4.** Method according to one of the preceding claims, wherein the alignment angle between the at least two bone parts is determined or estimated exclusively on the basis of reconstructed tomosynthesis image data, in particular slice images.

**5.** Method according to one of claims 1 to 3, wherein further projection images are used to determine or estimate the alignment angle between the at least two bone parts.

**6.** Method according to one of the preceding claims, wherein the distance (s) between two images of the plurality of partially spatially overlapping projection images is constant and less than or equal to half of the object width (d') which can be detected by an opening angle $\alpha$ of an x-ray source (3) of the recording system.

**7.** Method according to one of the preceding claims, wherein the elongated bone system is formed of a femur (6) and the alignment angle is formed of an antetorsion angle (13).

**8.** Method according to one of the preceding claims, wherein the elongated bone system is formed of a spinal column segment (8) and the alignment angle is formed of an angle between two vertebral bodies.

**9.** Method according to one of the preceding claims, wherein the translational movement is carried out by means of in particular automatically controlled rollers (23) of the mobile x-ray device along a floor.

**10.** Method according to one of the preceding claims, wherein the translational movement is carried out along at least one rail.

**11.** X-ray recording device for carrying out a method according to one of claims 1 to 9, which is embodied in particular as a mobile C-arm x-ray device (21), having

• a recording system held on a C-arm (20) and comprising an x-ray source (3) and an x-ray detector (2),

• a system controller (24) for controlling the recording of a plurality of partially spatially overlapping projection images by means of the recording system during a translational movement of the x-ray device, wherein the distance between two images of the plurality of partially spatially overlapping projection images differs as a function of a recording position along the elongated bone system by relevant regions being recorded with a small distance and less relevant with a larger distance, and
• an image processing unit (25) and a calculation unit (26) for reconstructing tomosynthesis image data, in particular slice images, and determining the alignment angle.

12. X-ray recording device according to claim 11, which is embodied as a mobile C-arm x-ray device (21), having a trolley (22) which can be moved automatically on rollers (23), on which the C-arm (20) is arranged.

## Revendications

1. Procédé de détermination d'un alignement entre au moins deux parties d'os d'un système d'os s'étendant en longueur d'un patient, comprenant les stades suivants :

    • enregistrement d'une pluralité d'images de projection se superposant dans l'espace, au moins en partie, par un système d'enregistrement d'un appareil à rayons X, notamment d'un appareil à rayons X mobile, pendant un mouvement de translation de l'appareil à rayons X ou du système d'enregistrement en direction de ou parallèlement à un axe longitudinal du système d'os (stade 10), dans lequel la distance entre respectivement deux images de la pluralité d'images de projection se superposant dans l'espace, au moins en partie, est différente en fonction d'une position d'enregistrement le long du système d'os s'étendant en longueur, par le fait que des parties pertinentes sont enregistrées à une distance petite et des parties moins pertinentes à une distance plus grande,
    • reconstruction de données d'image de tomosynthèse, notamment d'images de couche, des parties d'os, à partir des images de projection enregistrées (stade 11),
    • détermination ou estimation d'un angle d'alignement entre les au moins deux parties d'os, à l'aide au moins en partie des données d'images de tomosynthèse reconstruites et/ou des images de projection (stade 12).

2. Procédé suivant la revendication 1, que l'on effectue au moyen d'un appareil de rayons X à arceau en C mobile.

3. Procédé suivant l'une des revendications précédentes, dans lequel, pendant l'enregistrement et le mouvement de translation de l'appareil de rayons X, se produit en outre un mouvement de rotation du système d'enregistrement.

4. Procédé suivant l'une des revendications précédentes, dans lequel on détermine ou on évalue l'angle d'alignement entre les au moins deux parties d'os, à l'aide exclusivement de données d'image de tomosynthèse reconstruites, notamment d'images de couche.

5. Procédé suivant l'une des revendications 1 à 3, dans lequel on utilise d'autres images de projection pour la détermination ou l'évaluation de l'angle d'alignement entre les au moins deux parties d'os.

6. Procédé suivant l'une des revendications précédentes, dans lequel la distance (s), entre respectivement deux images de la pluralité d'images de projection se superposant dans l'espace, au moins en partie, est inférieure ou égale à la moitié de la largeur (d') d'objet pouvant être détectée par un angle α d'ouverture d'une source (3) de rayons X du système d'enregistrement.

7. Procédé suivant l'une des revendications précédentes, dans lequel le système d'os s'étendant en longueur est formé par un fémur (6) et l'angle d'alignement par un angle (13) d'antétorsion.

8. Procédé suivant l'une des revendications précédentes, dans lequel le système d'os s'étendant en longueur est formé par une partie (8) de la colonne vertébrale et l'angle d'alignement par un angle entre deux corps vertébraux.

9. Procédé suivant l'une des revendications précédentes, dans lequel le déplacement en translation s'effectue au moyen de roulettes (23), notamment commandées automatiquement, de l'appareil de rayons X mobile, le long d'un plancher.

10. Procédé suivant l'une des revendications précédentes, dans lequel le mouvement en translation s'effectue le long d'au moins un rail.

11. Dispositif de radiographie pour effectuer un procédé suivant l'une des revendications 1 à 9, qui est constitué notamment sous la forme d'un appareil (21) de rayons X à arceau en C mobile, comportant

    • un système d'enregistrement fixé à un arceau (20) en C et ayant une source (3) de rayons X et un détecteur (2) de rayons X,

• une commande (24) de système pour commander l'enregistrement d'une pluralité d'images de projection se superposant dans l'espace, au moins en partie, par le système d'enregistrement pendant un déplacement en translation de l'appareil à rayons X, dans lequel la distance, entre respectivement deux images de la pluralité d'images de projection se superposant dans l'espace, au moins en partie, est différente en fonction d'une position d'enregistrement le long du système d'os s'étendant en longueur, par le fait que l'on enregistre des parties pertinentes à une distance plus petite et des parties moins pertinentes à une distance plus grande, et

• une unité (25) de traitement d'image et une unité (26) de calcul pour la reconstruction de données d'image de tomosynthèse, notamment d'images de couche, et la détermination de l'angle d'alignement.

12. Dispositif radiographique suivant la revendication 11, qui est constitué sous la forme d'un appareil (21) de rayons X à arceau en C mobile, comportant un chariot (22) d'appareil, qui peut se déplacer automatiquement sur des roulettes (23), et sur lequel est disposé l'arceau (20) en C.

# FIG 1

# FIG 2

# FIG 3

# FIG 4

# FIG 5

FIG 6

## FIG 7

## FIG 8

## FIG 9

```
┌─────────────────────────────┐    ┌──────────────┐    ┌──────────────┐
│  □ □ □ □  · · ·  □           │    │ Rekonstruktion│    │ Bestimmung   │
│                             │ →  │ 3D-Bilder, z.B.│ → │ Ausrichtungs-│
│ Aufnahme einer Vielzahl von │    │ Schichtbild   │    │ winkel       │
│ Bildern während Translation │    │              │    │              │
└─────────────────────────────┘    └──────────────┘    └──────────────┘
              10                          11                  12
```

## FIG 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015201067 A1

- US 20120257714 A1